# EUROPÄISCHE PATENTSCHRIFT

(11) **EP 3 532 185 B1**
(45) Veröffentlichungstag und Bekanntmachung des Hinweises auf die Patenterteilung: **19.08.2020**
(21) Anmeldenummer: 17798133.9
(22) Anmeldetag: 27.10.2017
(51) Int. Cl.: B01D 39/16, B01D 39/08, B01D 39/18, A61B 50/30

(54) **MEHRLAGIGER FILTER ODER STERILVERPACKUNG, HERSTELLUNGSVERFAHREN UND VERWENDUNG DES FILTERS**
MULTILAYER FILTER OR STERILE PACKING, MANUFACTURING PROCESS AND USE OF THE FILTER
FILTRE MULTICOUCHE OU EMBALLAGE STÉRILE, PROCÉDÉ DE PRODUCTION ET UTILISATION DU FILTRE

(30) Priorität: 27.10.2016 DE 102016120530
(43) Veröffentlichungstag der Anmeldung: 04.09.2019
(73) Patentinhaber: Aesculap AG, 78532 Tuttlingen (DE)
(72) Erfinder: SCHUSTER, Stefan, 79865 Grafenhausen (DE)
(74) Vertreter: Winter, Brandl, Fürniss, Hübner, Röss, Kaiser, Polte - Partnerschaft mbB
(86) Internationale Anmeldenummer: PCT/EP2017/077647
(87) Internationale Veröffentlichungsnummer: WO 2018/078117

(56) Entgegenhaltungen:
- WO-A1-94/19180
- WO-A1-2012/161981
- DE-T2- 69 434 602

## Beschreibung

### Gebiet der Erfindung

Die vorliegende Erfindung betrifft einen mehrlagigen Filter für einen medizinischen Sterilisationsbehälter oder eine Sterilverpackung bestehend aus oder ausgerüstet mit dem mehrlagigen Filter, ein Verfahren zum Herstellen desselben und die Verwendung des mehrlagigen Filters in einem medizinischen Sterilisationsbehälter oder in einer medizinischen Sterilverpackung. Der mehrlagige Filter bzw. die daraus gebildete oder diesen aufweisende Sterilverpackung hat eine Kernschicht aus einem Aramidgewebe, die vollständig (beidseitig) von wenigstens einer Außenschicht aus einem anderen Material, vorzugsweise einem Filtermaterial, z.B. PTFE nach dem Sandwichprinzip (mindestens dreilagig) umschlossen wird.

### Hintergrund der Technik

Sterilisationsfilter (auch Sterilfilter oder Filtereinheiten) finden vor allem Anwendung bei medizinischen Sterilisationsbehältern (auch Sterilcontainer oder Sterilbehälter) zur Aufbewahrung von chirurgischem Besteck, chirurgischem Material oder anderem Sterilgut. Sterilisationsfilter zur Sterilfiltration für medizinische Sterilisationsbehälter sind üblicherweise in Form einer flachen, beispielsweise kreisförmigen Filterscheibe ausgebildet. Die Filterscheibe weißt dabei Fluidaustauschöffnungen (auch Poren genannt) auf, die den Austausch von Fluiden, insbesondere Gasen, zwischen der Umgebung und dem Inneren des medizinischen Sterilisationsbehälters ermöglicht. Der (medizinische) Sterilisationsfilter wird dabei beispielsweise in einer Filterhalterung gehalten, die an dem medizinischen Sterilisationsbehälter angebracht/ausgebildet ist. Dabei klemmt die Filterhalterung den Sterilisationsfilter zwischen einer ersten und einer zweiten Haltefläche oder einem ersten und einem zweiten Halterahmen ein.

Medizinische Sterilisationsbehälter werden im Allgemeinen dazu genutzt, chirurgisches Besteck oder Material (z.B. auch künstliche Implantate) zu sterilisieren, zu transportieren und zu lagern. Damit während des Sterilisiervorgangs, vorzugsweise in einem Autoklav, Heißdampf in den medizinischen Sterilisationsbehälter hineingelangen kann, wird eine Öffnung in der Behälterwand benötigt. Damit jedoch keine Keime, Bakterien oder dergleichen nach dem Sterilisieren in den Behälter über die Öffnung gelangen können, wird diese bereits vor dem Sterilisationsvorgang mit dem vorstehend erwähnten Sterilisationsfilter verschlossen/abgedeckt. Der Sterilisationsfilter lässt einen Fluidaustausch zu, lässt jedoch keine in den medizinischen Sterilisationsbehälter eindringen. In anderen Worten ausgedrückt, wird die Öffnung des medizinischen Sterilisationsbehälters durch eine Filtereinheit verschlossen, die viele kleine Fluidaustauschöffnungen/Poren aufweist, die einen Austausch von Fluiden, bzw. dessen Molekülen erlauben, aber das Eindringen von Keimen, Bakterien oder ähnlichem nach dem Sterilisationsvorgang unterbinden.

### Stand der Technik

Die für sterile Filter von Sterilisationsbehältern oder zur Sterilverpackung von Sterilgut verwendeten Materialien bestehen gemäß einem allgemein bekannten Stand der Technik aus Zellulose, Polypropylen (z.B. Kimguard ™/ Kimberly-Clark), Polyethylen (Tyvec®/DuPont) oder PTFE (z.B. Teflon ®, DuPont). In der Regel kommen die Materialien als verflochtene Fasern, gestreckte Folien, oder in gesinterter Form bei sterilen Filtern bzw. Sterilverpackungen zum Einsatz. Polytetrafluorethylen (Kurzzeichen PTFE, gelegentlich auch Polytetrafluorethen) ist ein unverzweigtes, linear aufgebautes, teilkristallines Polymer aus Fluor und Kohlenstoff. Umgangssprachlich wird dieser Kunststoff oft mit dem Handelsnamen Teflon der Firma DuPont bezeichnet. Weitere häufig verwendete Handelsnamen anderer Hersteller von PTFE sind Dyneon PTFE (ehemals Hostaflon) und Gore-Tex für PTFE-Membranen. DE 694 34 602 T2 offenbart eine medizinische Steriltuchverpackung umfassend ein äußeres Tuch, das aus einer ersten Sterilisationslage hergestellt ist und das auf einem inneren Tuch liegt, welches aus einer zweiten Sterilisationslage hergestellt ist, wobei jede Lage voneinander unabhängig ist. Die Sterilisationslagen können an ihrem äußeren Rand miteinander verbunden sind.

Da sterile Filter in Sterilisationsbehältern zur Sterilisation von chirurgischem Besteck oder Material oder auch als Verpackung von Sterilgut verwendet werden, kann es vorkommen, dass der sterile Filter in unbeabsichtigten Kontakt mit chirurgischem Besteck kommt und somit ein Defekt in dem Filter entstehen kann. Chirurgisches Besteck, wie zum Beispiel Messer, Skalpelle, Klemmen, Klammern, Nadeln, Sägen, Kanülen, Nägel, Schraubendreher, scharfe Löffel, Pinzetten, Scheren, Meißel oder Bohrer, kann Schneiden und/oder Spitzen aufweisen, die in den Filter mechanisch eindringen und diesen beschädigen können. Eine Beschädigung oder ein Defekt liegt in dem Fall vor, wenn durch das Filtermaterial hindurch eine Öffnung in den Sterilfilter eingebracht wird, die größer ist, als eine Fluidaustauschöffnung. Ein beschädigter Filter kann nach dem Sterilisationsprozess somit keine Sterilität im Sterilisationsbehälter gewährleisten. In anderen Worten ausgedrückt, ist der Nachteil der bereits bekannten Materialen, dass sie gegenüber den darin verpackten Gegenständen bzw. dem im Sterilationsbehälter gelagerten Besteck nur einen geringen mechanischen Schutz/Wiederstand bieten/leisten (z.B. Durchstechsicherheit). Und bei einer Beschädigung des Filters Verunreinigungen in den Sterilbehälter gelangen können.

### Zusammenfassung der Erfindung

In Anbetracht dieser Situation ist es die Aufgabe der vorliegenden Erfindung, einen Filter bzw. eine Sterilverpackung bereitzustellen, der/die bei unbeabsichtigtem mechanischem Kontakt mit dem Sterilgut, beispielsweise chirurgischem Besteck oder ähnlichem nur schwer zu beschädigen ist. Vorzugsweise soll durch einen durchstechsicheren Filter bzw. eine durchstechsichere Sterilverpackung ein sicherer, steriler Medienaustausch in einem medizinischen Sterilisationsbehälter oder in einer Sterilgutverpackung, sowie die sichrer Lagerung des darin verpackten Sterilguts gewährleistet werden. Dabei sollte das Filter-/Verpackungsmaterial vorzugsweise als flexibles Folienmaterial vorliegen. Des Weiteren soll der Inhalt vor Verunreinigungen geschützt werden, die durch eine Beschädigung des Filters oder dergleichen entstehen können.

Diese Aufgabe wird durch einen mehrlagigen, vorzugsweise sandwichartig aufgebauten Filter für einen/eines medizinischen Sterilisationsbehälter(s) oder eine Sterilverpackung von Sterilgut mit den Merkmalen der unabhängigen Ansprüche gelöst. Vorteilhafte Ausgestaltungen der Erfindung sind Gegenstand der Unteransprüche.

Der Grundgedanke der Erfindung besteht darin, die Kernschicht, vorzugsweise Mittelschicht des (sandwichartig aufgebauten) Filters/Sterilverpackung (-Material) aus einem Aramidgewebe auszubilden. Die Kernschicht ist insbesondere gemäß einem Sandwichaufbau (mindestens dreilagig) von wenigstens einer Außenschicht aus mindestens einem anderen (Filter-)Material umgeben bzw. bevorzugt vollständig (auch randseitig) umschlossen, vorzugsweise PTFE. Durch einen vollständigen Einschluss des Aramidgewebes können somit auch keine Aramidgewebefasern, die sich evtl. lösen, in den Sterilbehälter gelangen.

Alle Schichten des Filters, also sowohl die bevorzugt mindestens zwei äußeren Schichten aus einem (Filter-)Material, als auch die Kernschicht aus Aramidgewebe, bilden/weisen Fluidaustauschöffnungen (Poren) auf, die den Austausch von Fluiden, insbesondere Gasen, zwischen der Umgebung und dem Inneren des Sterilbehälters/der Sterilverpackung ermöglichen und eine Durchstechsicherheit herstellen. Konkreter ausgedrückt, übernehmen die bevorzugt beiden äußeren Schichten die eigentliche Filterfunktion, wohingegen das (dazwischen angeordnete) Aramidgewebe (ausschließlich) die Durchstechsicherheitsfunktion übernimmt, wobei dessen Poren gegenüber den Poren der bevorzugt beiden äußeren Schichten (deutlich) größer sein kann. Hier sei darauf hingewiesen, dass die Filterfunktion der beiden äußeren Schichten bevorzugt infolge ihrer jeweiligen Porengröße und/oder deren jeweilige 3-dimensionalen (Schwamm-)Struktur erzielt werden kann. D.h., insbesondere im letzteren Fall können die Poren der äußeren Schichten sogar größer sein als der kleinste Keim und trotzdem können auch die kleinsten Keime die Schichten nicht passieren.

Somit können nur Fluide wie Luft oder Wasserdampf die dadurch gebildete bevorzugt dreilagige Membran passieren, größere Partikel wie zum Beispiel Bakterien und Sporen werden zumindest durch die äußeren Schichten aus Filtermaterial zurückgehalten. Erfolgt im Fall einer Sandwichbauart eine mechanische Einwirkung (Einstechen/Einschneiden) auf eine der beiden äußeren Schichten, wird diese ggf. verletzt/beschädigt, derart, dass diese die Filterfunktion verliert. Indessen bleibt die Filterfunktion der anderen äußeren Schicht erhalten, da das die beiden äußeren Schichten trennende Aramidgewebe ein Durchstechen/Durchschneiden der gesamten Filter-/Verpackungsmembran bis zur anderen äußeren Schicht verhindert und damit zumindest deren Filterfunktion aufrecht erhält/schützt.

Die Fluidaustauschöffnungen/Poren können dabei in allen Lagen des mehrlagigen Filters/Sterilverpackung im Wesentlich die gleiche Größe bzw. den gleichen Durchmesser aufweisen oder aber die Fluidaustauschöffnungen/Poren der mittleren Durchstech-Schutzlage/Schutzschicht (Aramidgewebe) können größer sein als die der äußeren Filter-/Verpackungslagen bzw. -schichten.

Die einzelnen Lagen/Schichten können in einer Ausführungsform als Filterscheiben ausgebildet sein. In anderen Worten ausgedrückt kann der Filter im Querschnitt beispielsweise aus wenigstens drei Lagen bzw. aus wenigstens drei einzelnen Filterscheiben bestehen. Die wenigstens eine erste, äußere (untere) Lage bzw. Filterscheibe kann dabei eines der bekannten Filtermaterialen aus der Gruppe Zellulose oder Kunststoff bzw. Polymer, bevorzugt Polypropylen (z.B. Kimguard ™/ Kimberly-Clark), Polyethylen (Tyvec®/DuPont) oder PTFE (z.B. Teflon ®, DuPont) sein, die als verflochtene Faser, gestreckte Folie, oder in gesinterter Form vorliegen. Die zweite Schicht bzw. Filterscheibe (die mittlere Schicht/die Kernschicht) besteht aus dem genannten Aramidgewebe. Die darauf liegende, wenigstens eine zweite, äußere (oberste) Schicht bzw. Filterscheibe besteht auch wieder aus einem der Filtermaterialien der oben erwähnten Gruppe der ersten Schicht. Das Aramidgewebe ist in diesem bevorzugten Fall sozusagen in die beiden anderen Materialien eingebettet oder anders gesagt es wird von ihnen (sandwichartig) umhüllt. Dabei schließen die beiden äußeren Schichten bzw. Filterscheiben die Kernschicht vollkommen ein.

An dieser Stelle sein nochmals ausdrücklich darauf hingewiesen, dass der Begriff "sandwichartig" mindestens drei Lagen voraussetzt, von denen wenigstens eine Lage von zwei weiteren Lagen dazwischen eingeschlossen wird. Zweilagige Verbundmaterialien sind demnach nicht "sandwichartig". Des Weiteren sind die Begriffe Lage, Schicht und Scheibe als 3-dimensionale (Schwamm) Struktur zu verstehen, die auf Grund des Verhältnisses der Oberfläche zu der Dicke aus Gründen der Einfachheit diese Bezeichnungen tragen.

Die Kernschicht aus Aramidgewebe kann in einer anderen Ausführungsform auch mit einem der Materialien aus der Gruppe von Zellulose oder Kunststoff bzw. Polymer, insbesondere Polypropylen und/oder PTFE direkt beschichtet (Tauchbeschichten, Besprühen und dergleichen Direktbeschichtungen) sein, vorzugsweise bedampft. Durch diesen Aufbau wird nicht nur die Oberfläche der Kernschicht vollständig eingeschlossen/bedeckt sondern auch die Oberfläche der Fluidaustauschöffnungen in der Kernschicht ist somit beschichtet (d.h. das Filtermateril bedeckt nicht nur die beiden voneinander abgewandten Flachseiten der Aramidgewebeschicht sondern dringt in deren Poren ein). Damit ist an dem Aramidgewebe, also der Kernschicht die ganze Oberfläche vollständig mit einem der oben genannten Materialien beschichtet. Dadurch schützt die direkte Beschichtung das Aramidgewebe nicht nur vor UV-Strahlung und chemischer Beeinflussung durch Säuren oder ähnlichem, sondern vermeidet auch, dass die kürzeren Fasern des Aramidgewebes sich von dem Gewebe ablösen können und in den sterilen Bereich gelangen. Ein weiterer Vorteil bei direkter Beschichtung durch das Filtermaterial ist, dass man die Größe bzw. den Durchmesser der Fluidaustauschöffnungen direkt durch die Beschichtungsdauer kontrollieren kann. Durch eine solche Beschichtung werden selbstständig Fluidaustauschöffnungen in der Beschichtung gebildet, die sich an die Fluidaustauschöffnungen in dem Aramidgewebe anpassen.

Durch einen derartigen mehrlagigen (bevorzugt mindestens dreilagigen) Filter/Sterilverpackung wird eine mechanische Belastbarkeit sichergestellt, die sich dadurch äußert, dass der Filter/Sterilverpackung im Wesentlichen durchstechsicher(er) ist und von einem scharfen oder spitzen chirurgischem Instrument nicht/schlecht durchstochen oder beschädigt werden kann. Andererseits wird durch den mehrlagigen (bevorzugt mindestens dreilagigen) Aufbau auch verhindert, dass Aramidfasern aus dem Aramidgewebe freigesetzt werden können und in den sterilen Raum gelangen. Gleichzeitig bleibt der Filter/Sterilverpackung, der/die aus mehreren Lagen gebildet ist, elastisch und unempfindlich gegenüber Druck- und Biegebeanspruchungen. Durch einen mehrlagigen Filter/Sterilverpackung wie oben beschrieben lassen sich zusätzlich längere Wartungsintervalle realisieren, wodurch ein Austausch nur noch selten erforderlich ist, was wiederum zu einer Kostenreduktion führt. Ein weiterer Vorteil der bereits genannten Filtermaterialien aus der Gruppe Zellulose oder Kunststoffe, insbesondere Polypropylen, oder PTFE ist, dass diese Materialien das Aramidgewebe vor UV-Strahlung schützen. UV-Strahlung verursacht bei Aramidgewebe zunächst eine sichtbare Verfärbung vom ursprünglichen hellen Gelb in einen bronzebraunen Farbton. Nach längerer Einwirkung der UV-Strahlung verliert die Faser bis zu 75 % an Festigkeit.

In anderen Ausführungsformen kann der Filter/Sterilverpackung auch mehr als drei Lagen im Querschnitt aufweisen, zum Beispiel mehrere Aramidschichten oder mehrere Schichten aus den bekannten Filtermaterialien, bevorzugt Kunststoff, die zwischen den Aramidschichten angeordnet sind und/oder auch die wenigstens eine Aramidschicht umhüllen. In wieder einer anderen Ausführungsform kann der mehrlagige Filter/Sterilverpackung auch nur aus zwei Schichten bzw. Filterscheiben gebildet werden. Bei einer Ausführungsform mit zwei Filterscheiben ist dabei die Aramidschicht auf der dem Sterilisationsgut abgewandten Seite und die andere Filterschicht auf der dem Sterilisationsgut zugewandten Seite.

Aromatisches Polyamid (Aramid) ist auch unter dem Namen Kevlar® bekannt. Aramidfasern besitzen eine hohe spezifische (gewichtsbezogene) Festigkeit, niedrige Dichte, hohe Schlagzähigkeit, gute Wärmebeständigkeit und Dimension-Stabilität, gute Schwingungsdämpfung und ein hohes Arbeitsaufnahmevermögen. Aramidfasern weisen eine gute Beständigkeit gegen Lösemittel, Kraftstoffe, Schmiermittel, Salzwasser etc. auf; von einigen starken Säuren und Laugen werden Aramidfasern indessen angegriffen. Sie sind widerstandsfähig gegen den Angriff von Pilzen und Bakterien. Aramidfasern finden eine breite Anwendung. Sie sind vor allem durch ihre Anwendung in Sicherheitsbekleidung bekannt (Splitterwesten, Schnittschutzhandschuhe). Die Fasern weisen eine hohe mechanische Festigkeit auf.

Da die einzelnen Aramidfasern kurz sind, können sie sich unter Umständen von dem Aramidgewebe ablösen. Um zu verhindern, dass diese Fasern in den medizinischen Sterilisationsbehälter oder in den sterilisierten Bereich einer Sterilverpackung gelangen, ist das Aramidgewebe von einem weiteren Filtermaterial aus einem der bereits bekannten Materialien umhüllt, sozusagen ist der Filter mehrlagig, d.h. in diesem Fall sandwichartig aufgebaut. In dem bevorzugten Ausführungsbeispiel wird der Filter/Sterilverpackung dreilagig aufgebaut, wobei die mittlere Lage aus dem Aramidgewebe besteht, das von beiden Seiten durch Filtermaterial aus der oben genannten bekannten Gruppe umschlossen wird, vorzugsweise aus PTFE. Das Aramidgewebe in dem mehrlagigen Filter/Sterilverpackung mit einem Aramidkern ist somit flexibel, ggf. elastisch und/oder unempfindlich gegenüber Druck- und Biegebeanspruchungen.

Der Filter/Sterilverpackung, genauer gesagt, die bevorzugt wenigstens zwei äußeren Schichten bzw. Filterscheiben, können in einem Ausführungsbeispiel einen größeren Flächendurchmesser als die Kernschicht aufweisen und an ihren äußeren Rändern der Scheiben miteinander verklebt sein oder die äußeren Schichten können auch direkt an die Kernschicht geklebt sein. Ein solcher Aufbau ist einfach zu realisieren, was die Herstellungskosten senkt. Durch diesen Aufbau ist die Kernschicht bevorzugt vollständig von den beiden Außenschichten eingeschlossen.

Die Kernschicht und die Außenschichten des Filters/Sterilverpackung weisen Fluidaustauschöffnungen/Poren auf, wie dies vorstehend bereits ausgeführt wurde. In einer Ausführungsform können die Fluidaustauschöffnungen der Kernschicht vorzugsweise größere Durchmesser aufweisen als die Fluidaustauschöffnungen der Außenschicht. Das hat den Vorteil, dass man ein gröberes und somit günstigeres durchstechsicheres Aramidgewebe im Kern verwenden kann, und ein feineres Material, also ein Material mit kleineren Fluidaustauschöffnungen, als Außenschicht.

Die Fluidaustauschöffnungen der wenigstens einen Außenschicht können in einer Ausführungsform kleiner sein, als der Durchmesser von Fasern des Aramidgewebes. Dadurch, dass die Fasern somit nicht durch die Außenschicht wandern können, wird sichergestellt, dass die Fasern nicht in den sterilisierten Raum kommen und diesen verunreinigen.

Der mehrlagige Filter kann im Wesentlichen kreisförmig, quadratisch oder rechteckig ausgebildet sein. Er kann auch jede andere Form aufweisen, um an eine Filterhaltevorrichtung in einem medizinischen Sterilisationsbehälter angepasst zu werden. Des Weiteren sind auch noch andere Formen realisierbar wie zum Beispiel Taschen oder Beutel aus diesem mehrlagigen Filter-Verbundmaterial, um chirurgisches Besteck oder Material zu verpacken. Vorzugsweise ist der Filter zwar ein Dauerfilter, er kann aber auch als Einwegfilter konzipiert sein.

Des Weiteren wird ein Verfahren zum Herstellen eines mehrlagigen Filters für einen/eines medizinischen Sterilisationsbehälter(s) bzw. einer mehrlagigen Sterilverpackung vorgestellt. Das Verfahren beinhaltet, das Umschließen einer Kernschicht aus Aramidgewebe mit mindestens einem anderen (Filter-)Material, wobei das mindestens eine andere Material aus der Gruppe Zellulose oder Kunststoff, bevorzugt aus Polypropylen (z.B. Kimguard ™/ Kimberly-Clark), Polyethylen (Tyvec®/DuPont) oder PTFE (z.B. Teflon ®, DuPont) ist, oder aus einem anderen (Filter-) Material mit ähnlichen/vergleichbaren mechanischen/chemischen Eigenschaften besteht.

Das Verfahren zum Herstellen des Filters kann ferner beinhalten, das Aneinanderkleben von zwei separaten Filterscheiben bzw. äußeren Filterschichten. Die Filterscheiben/Filterschichten sind vorzugsweise aus einem Material der Gruppe Zellulose oder Kunststoff, bevorzugt aus Polypropylen (z.B. Kimguard ™/ Kimberly-Clark), Polyethylen (Tyvec®/DuPont) oder PTFE (z.B. Teflon ®, DuPont). Dabei werden die Filterscheiben/Filterschichten (unter Einschluss des Aramidgewebes) an ihrem äußeren Rand verklebt. Unter äußerem Rand ist hierbei die (Rahmen-)Fläche zu verstehen, die auf jener Seite liegt, die der anderen Filterscheibe/Filterschicht zugewandt ist und sich in der Nähe zu dem Außenumfang der Filterscheibe/Filterschicht befindet, wobei unter Nähe zum Außenumfang vorzugsweise eine Distanz zum Außenumfang der jeweiligen Filterscheibe/Filterschicht zu verstehen ist, die weniger als neunzig Prozent des Radius/Querschnittsabmessung beträgt, bevorzugt weniger als 50 Prozent und besonders bevorzugt weniger als 10 Prozent des Radius/Querschn ittsabmessung.

Eine anderes Herstellungsverfahren beinhaltet das Umschließen der Kernschicht aus Aramidgewebe durch (direktes) Beschichten, bevorzugt durch Bedampfen, der Kernschicht, besonders bevorzugt mit einem Material der Gruppe Zellulose oder Kunststoff, bevorzugt aus Polypropylen (z.B. Kimguard ™/ Kimberly-Clark), Polyethylen (Tyvec®/DuPont) oder PTFE (z.B. Teflon ®, DuPont), wobei insbeondere durch das Bedampfen das (Filter-)Material direkt auf dem Aramidgewebe aufgetragen wird. Somit ist jede Oberfläche der Zwischenschicht (Aramidgewebe) vollständig mit dem (Filter-) Material beschichtet.

Eine medizinische Sterilverpackung weist in einer bevorzugten Ausführungsform einen mehrlagigen Filter auf, bestehend aus einer Kernschicht oder Kernlage aus einem Aramidgewebe als Durchstechsicherung. Die Kernschicht oder Kernlage, ist von wenigstens einer ersten Außenschicht oder Außenlage aus einem Filtermaterial an deren einer Seite und/ oder von wenigstens einer zweiten Außenschicht/ oder Außenlage aus einem Filtermaterial an deren anderer Seite (über den Umfangsrand der Kernschicht hinaus) vollständig umschlossen.

Die vorliegende Erfindung wird nachstehend anhand bevorzugter Ausführungsformen unter Bezugnahme auf die begleitenden Figuren näher erläutert.

### Figurenbeschreibung

Fig. 1 zeigt einen medizinischen Sterilisationsbehälter.
Fig. 2 zeigt eine Filterhaltevorrichtung für einen medizinischen Sterilisationsbehälter.
Fig.3 zeigt das Herstellungsverfahren einer ersten Ausführungsform eines mehrlagigen Filters.
Fig.4 zeigt den Aufbau der ersten Ausführungsform eines mehrlagigen Filters in Querschnittsansicht.
Fig.5 zeigt den Aufbau einer zweiten Ausführungsform eines mehrlagigen Filters in Querschnittsansicht.
Fig. 6 zeigt das Herstellungsverfahren einer dritten Ausführungsform eines mehrlagigen Filters.
Fig.7 zeigt den Aufbau einer dritten Ausführungsform eines mehrlagigen Filters in Querschnittsansicht.

### Detaillierte Beschreibung

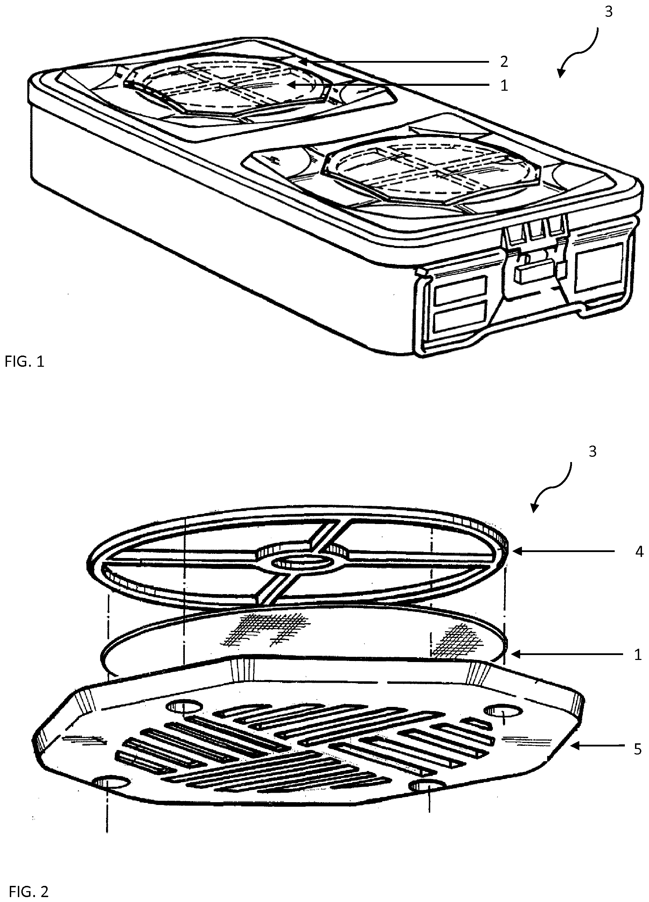

In Fig. 1 ist ein mehrlagiger Filter 1 in einer Haltevorrichtung 2 eines medizinischen Sterilisationsbehälters 3 dargestellt. Hierbei ist der Filter 1 als annähernd kreisrunde Scheibe ausgebildet und in der rahmenartigen Haltevorrichtung 2 eingefasst, vorzugsweise eingeklemmt, die wiederum an dem medizinischen Sterilisationsbehälter 3 befestigt ist.

In Fig. 2 ist der Aufbau der Filterhaltevorrichtung 2 für einen mehrlagigen Filter 1 in einem medizinischen Sterilisationsbehälter 3 dargestellt. Die Filterhaltevorrichtung 3 besteht aus einem fachwerkartig ausgebildeten Haltering 4 und einer gitterrost- oder lochplattenartig ausgebildeten Halteplatte 5, die zwischen sich den mehrlagigen Filter 1 halten/einspannen. Dadurch sind in der Filterhaltevorrichtung 3, sowohl im Haltering 4 als auch in der Halteplatte 5, Durchlassöffnungen entstanden, die einen Fluidaustauch zwischen dem Innenraum des Sterilisationsbehälters 3 und dessen Umgebung ermöglichen und den mehrlagigen Filter 1 gleichzeitig vor groben/großflächigen Krafteinflüssen schützen.

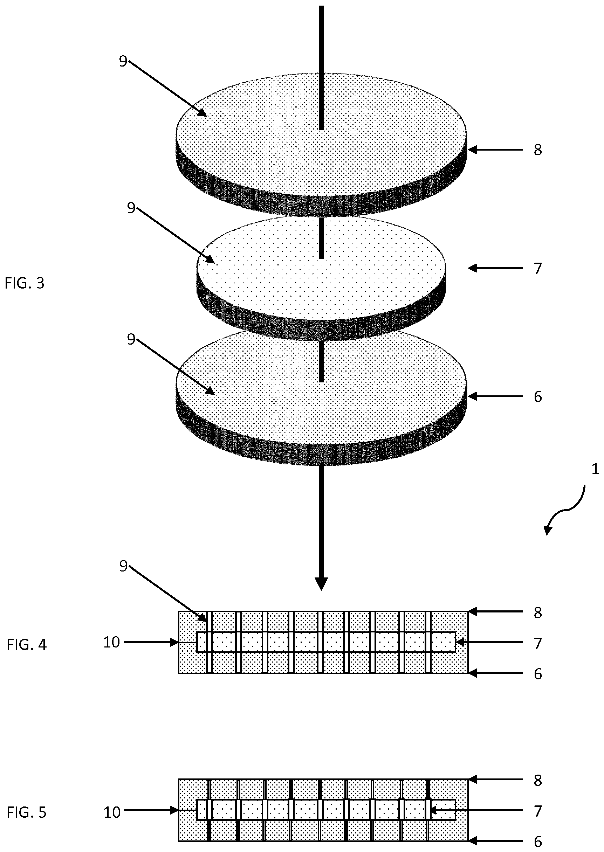

In Fig. 3 ist das Herstellungsverfahren sowie der konstruktive Aufbau eines mehrlagigen Filters 1 gemäß einer ersten Ausführungsform der vorliegenden Erfindung dargestellt. Demnach werden alle Elemente des mehrlagigen Filters 1 (Verbundwerkstoff/Verbundmembran) aufeinandergestapelt, so dass sie alle zentral längs einer imaginären Achse (in Membrandickenrichtung) angeordnet sind. Unter dem Begriff "Elemente" sind dabei die einzelnen Schichten bzw. Filterscheiben des mehrlagigen Filters 1 zu verstehen.

Der mehrlagige Filter 1 hat eine unterste/äußerste erste Schicht/Lage 6 aus einem Filtermaterial bevorzugt der Gruppe Zellulose oder Kunststoff, bevorzugt aus Polypropylen (z.B. Kimguard ™/ Kimberly-Clark), Polyethylen (Tyvec®/DuPont) oder PTFE (z.B. Teflon ®, DuPont). Die zumindest eine mittlere, zweite Schicht/Lage, nämlich die Kernschicht 7 besteht aus einem Aramidgewebe. Die oberste/äußerste dritte Schicht 8 ist wieder aus einem Material der Gruppe Zellulose oder Kunststoff, bevorzugt aus Polypropylen (z.B. Kimguard ™/ Kimberly-Clark), Polyethylen (Tyvec®/DuPont) oder PTFE (z.B. Teflon ®, DuPont). Dieser bevorzugte sandwichartige Aufbau ist exemplarisch und kann auch mehrere weitere Schichten/Lagen aus Aramid betreffen sowie auch mehrere Schichten/Lagen aus den bereits genannten Filtermaterialien oder Materialien mit ähnlichen (Filter-)Eigenschaften. Sämtliche Schichten/Lagen weisen Fluidaustauschöffnungen (Poren) 9 auf, wobei die erste und dritte Schicht/Lage 6, 8 Fluidaustauschöffnungsdurchmesser (Porengröße) aufweisen, die kleiner sind als der Faserdurchmesser des Aramidgewebes der zweiten Schicht 7.

In dem Herstellungsverfahren für die erste Ausführungsform des mehrlagigen Filters 1 werden die einzelnen Schichten/Lagen mittels eines Klebers oder mittels Ultraschallschweißen in der Nähe des Außenumfangs der beiden äußeren Schichten/Lagen 6, 8 verklebt und schließen somit die zweite Schicht 7, nämlich das Aramidgewebe zwischen sich (lose) ein.

Die beiden äußeren Schichten/Lagen aus geeignetem Filtermaterial bilden somit die eigentlichen Filtermembrane mit entsprechenden Filtereigenschaften, wohingegen die mittlere Schicht/Lage aus dem Aramidgewebe (ausschließlich) eine Durchstechsicherung darstellt. Wird demnach der Filter 1 an einer Flachseite durch mechanische Krafteinwirkung beschädigt und wird damit die Filtereigenschaft der einen äußeren Schicht/Lage aufgehoben/beeinträchtigt, bleibt durch die Zwischenlage aus Aramidgewebe die andere äußere Schicht/Lage unbeschädigt und damit deren Filterwirkung intakt.

In Fig. 4 ist der Querschnitt der ersten Ausführungsform des mehrlagigen Filters 1 gemäß der ersten Ausführungsform dargestellt. In dieser Ausführungsform weisen alle Fluidaustauschöffnungen/Poren 9 der Schichten den gleichen Fluidaustauschöffnungsdurchmesser/Porendurchmesser auf. Die Zwischenschicht aus Aramidgewebe, also die zweite Schicht 7, ist zwischen den beiden anderen, äußeren Schichten 6 und 8 eingebettet und vollständig umschlossen. Die Umfangsrand-seitigen Kontakt-/Verbindungsbereiche 10 der beiden äußeren Schichten/Lagen sind miteinander verklebt, können aber auch herstellungsbedingt verflochten, verstemmt, vernietet sein oder ähnliches. In dieser Ausführungsform sind nur die beiden äußeren Schichten/Lagen miteinander fest verbunden und die mittlere Schicht/Lage lose dazwischen aufgenommen. Die beiden äußeren Schichten/Lagen können aber auch an dem mittleren Filter miteinander fest verbunden (verklebt) sein. Es sind auch andere Ausführungsbeispiele des Verklebens naheliegend, die eine komplette Umschließung der Kernschicht beinhalten z.B. Verschweißen (bevorzugt Ultraschallschweißen)oder Verpressen.

In Fig. 5 ist eine zweite Ausführungsform eines mehrlagigen Filters 1 dargestellt, bei der die beiden äußeren Schichten/Lagen einen Fluidaustauschöffnungsdurchmesser bzw. Porendurchmesser aufweisen, der kleiner ist als der Fluidöffnungsaustauschdurchmesser der Kernschicht 7. Das Verfahren zu dessen Herstellung entspricht dabei dem ersten bevorzugten Ausführungsbeispiel.

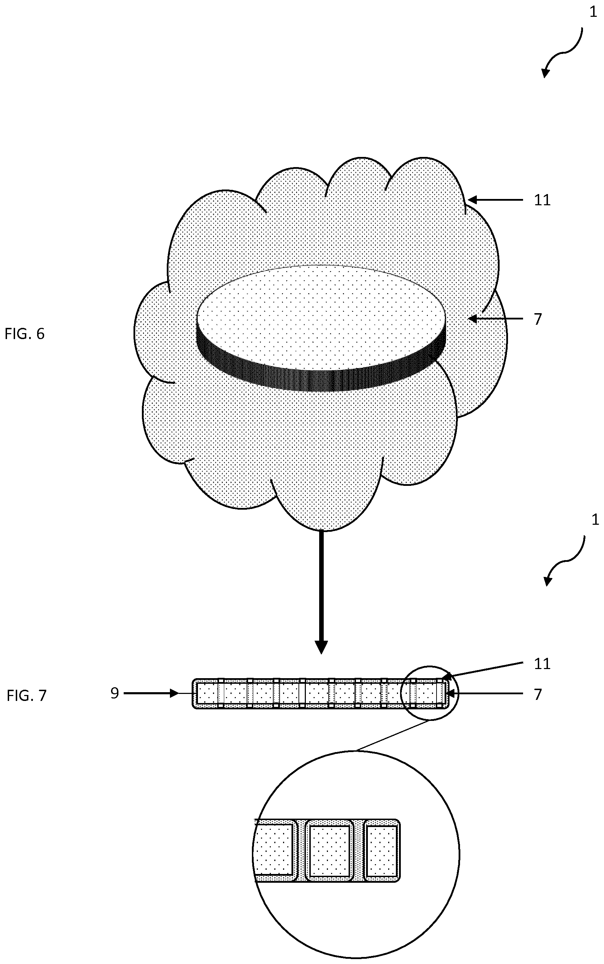

In Fig. 6 ist das Herstellungsverfahren einer dritten Ausführungsform eines mehrlagigen Filters 1 dargestellt. Dabei wird der Aramidkern, also die mittlere Schicht mit einem Beschichtungsmaterial 11 der Gruppe Zellulose oder Kunststoff, bevorzugt aus einem Polymer insbesondere Polypropylen (z.B. Kimguard ™/ Kimberly-Clark), Polyethylen (Tyvec®/DuPont) oder PTFE (z.B. Teflon ®, DuPont) beschichtet. Dies kann durch Kunststoff- und Pulverbeschichtung, Nasslackierung, Spritz-SinterVerfahren, elektrothermische Verfahren und bedampfen erfolgen.

In Fig. 7 ist der Querschnitt einer dritten Ausführungsform des mehrlagigen Filters 1 als Ganzes und in Detailansicht dargestellt. In dieser Ausführungsform sind alle Außenseiten (Ober- und Unterseite) des Aramidgewebes 7 und auch alle vom Aramidgewebe 7 gebildeten Fluidaustauschöffnungen/Poren 9 (poreninnenseitig) mit einem (Filter-)Material 11 der bereits bekannten Gruppe beschichtet. Hierbei gelangt die Beschichtung nicht nur auf die Schichtoberflächen bzw. Scheibenoberflächen des Aramidgewebes 7 sondern auch auf die inneren Oberflächen der Fluidaustauschöffnungen/Poren 9. Der mehrlagige Filter 1 gemäß dem dritten bevorzugten Ausführungsbeil der vorliegenden Erfindung wird in dieser Ausführungsform nicht aus drei einzelnen separaten Lagen gebildet sondern aus einer mittleren Lage 7, nämlich dem Aramidgewebe, und einer diese vollständig umhüllenden sowie deren Poren durchdringenden Beschichtung 11.

Die Erfindung betrifft zusammenfassend einen mehrlagigen Filter eines (medizinischen) Sterilbehälters oder eine (medizinische) Sterilverpackung jeweils bestehend aus einer Kernschicht aus einem Aramidgewebe, die von mindestens einer Außenschicht aus wenigstens einem anderen Material vorzugsweise aus PTFE und ganz besonders bevorzugt aus Teflon bedeckt oder vollständig umschlossen ist, ein Verfahren zur Herstellung des mehrlagigen (medizinischen) Filters eines Sterilbehälters oder zur Herstellung der (medizinischen) Sterilverpackung und die Verwendung einer Verbundmembran als mehrlagiger Filter eines (medizinischen) Sterilbehalters oder als (medizinische) Sterilverpackung, wobei die Verbundmembran aus einer Kernschicht aus einem Aramidgewebe, besteht, die von mindestens einer Außenschicht aus wenigstens einem anderen Material vorzugsweise aus PTFE und ganz besonders bevorzugt aus Teflon bedeckt oder vollständig umschlossen ist.

### Bezugszeichenliste

1. Mehrlagiger Filter
2. Filterhaltevorrichtung
3. medizinischer Sterilisationsbehälter
4. Haltering
5. Halteplatte
6. erste Schicht
7. zweite Schicht (Kernschicht, Aramidgewebe)
8. Dritte Schicht
9. Fluidaustauschöffnungen
10. Kontaktstelle
11. Beschichtung

## Patentansprüche

1. Mehrlagiger Filter (1) eines medizinischen Sterilisationsbehälters (3) oder eine medizinische Sterilverpackung, jeweils bestehend aus einer Kernschicht oder Kernlage (7) aus einem Aramidgewebe als Durchstechsicherung, die von wenigstens einer ersten Außenschicht oder Außenlage (6) aus einem Filtermaterial an deren einer Seite und von wenigstens einer zweiten Außenschicht/ oder Außenlage (8) aus einem Filtermaterial an deren anderer Seite vollständig umschlossen ist.

2. Filter (1) oder Sterilverpackung nach einem der vorgehenden Ansprüche, **dadurch gekennzeichnet, dass** alle Schichten/Lagen (6, 7, 8) des Filters (1) Fluidaustauschöffnungen oder Poren (9) aufweisen, die den Austausch von Fluiden, insbesondere Gasen, zwischen der Umgebung und dem inneren des medizinischen Sterilisationsbehälters (3) oder der Sterilverpackung ermöglichen.

3. Filter (1) oder Sterilverpackung nach einem der vorgehenden Ansprüche, **dadurch gekennzeichnet, dass** die wenigstens eine erste Außenschicht/Lage oder die wenigstens eine erste und zweite Außenschichten/Lagen (6, 8) aus einem Material der Gruppe Zellulose oder Kunststoff, bevorzugt aus Polypropylen, Polyethylen oder PTFE besteht/bestehen.

4. Filter (1) oder Sterilverpackung nach einem der vorgehenden Ansprüche, **dadurch gekennzeichnet, dass** die wenigstens eine erste und zweite Außenschichten (6, 8) einen größeren Flächendurchmesser als die Kernschicht (7) aufweisen und an ihrem äußeren, die Kernschicht (7) umgebenden Rand miteinander verklebt sind oder dass die wenigstens eine erste und zweite Außenschichten (6, 8) direkt an die Kernschicht (7) geklebt sind.

5. Filter oder Sterilverpackung nach einem der Ansprüche 2 bis 4, **dadurch gekennzeichnet, dass** die Fluidaustauschöffnungen oder Poren (9) der wenigsten einen ersten Außenschicht (6) oder der wenigstens einen ersten und zweiten Außenschichten (6, 8) kleiner sind als der Durchmesser von Fasern des Aramidgewebes (7).

6. Filter (1) oder Sterilverpackung nach einem der Ansprüche 1 bis 3, **dadurch gekennzeichnet, dass** die Kernschicht (7) mit einem Beschichtungsmaterial (11) aus der Gruppe von Zellulose, Polymer, Polypropylen und/oder PTFE beschichtet, vorzugsweise bedampft ist.

7. Filter (1) oder Sterilverpackung nach einem der vorgehenden Ansprüche, **dadurch gekennzeichnet, dass** die Kernschicht (7) und die wenigstens eine Außenschicht (6) oder die wenigstens eine erste und zweite Außenschichten (6, 8) Fluidaustauschöffnungen oder Poren (9) aufweisen, wobei die Fluidaustauschöffnungen oder Poren (9) der Kernschicht (7) vorzugsweise größere Durchmesser aufweisen als die Fluidaustauschöffnungen oder Poren (9) der wenigstens einen Außenschicht (6,8).

8. Verfahren zum Herstellen eines mehrlagigen Filters eines medizinischen Sterilisationsbehälters oder zum Herstellen einer medizinischen Sterilverpackung **gekennzeichnet durch** die folgenden Verfahrensschritte:
Bereitstellen einer Kernschicht oder Kernlage aus einem Aramidgewebe als Durchstechsicherung,
Bedecken der Kernschicht oder Kernlage an deren einen Seite als Innenseite mit wenigstens einer ersten Außenschicht oder Außenlage aus einem Filtermaterial und
Bedecken der Kernschicht oder Kernlage an deren anderen Seite als Außenseite mit wenigstens einer zweiten Außenschicht oder Außenlage aus einem Filtermaterial, so dass die Kernschicht oder Kernlage völlig umschlossen ist.

9. Verfahren nach Anspruch 8, **dadurch gekennzeichnet, dass** für den Fall, dass beide Seiten der Kernschicht oder Kernlage bedeckt werden, die erste und zweite Außenschichten aus zwei separaten Filterscheiben oder Membare gebildet werden, bevorzugt aus einem Material der Gruppe Zellulose oder Kunststoff, bevorzugt aus Polypropylen, Polyethylen oder PTFE, die an ihrem jeweils äußeren, die Kernschicht oder Kernlage umschließenden Rand unmittelbar miteinander verklebt werden.

10. Verfahren nach Anspruch 8, **dadurch gekennzeichnet, dass** für den Fall, dass beide Seiten der Kernschicht oder Kernlage bedeckt werden, die Kernschicht aus Aramidgewebe mit dem Material der ersten und zweiten Außenschichten beschichtet, vorzugssweise bedampft wird, wobei die erste und zweite Außenschichten bevorzugt aus einem Material der Gruppe Zellulose oder Kunststoff, bevorzugt aus Polypropylen, Polyethylen oder PTFE besteht.

11. Verwendung eines mehrlagigen Filters nach Anspruch 1 in einem medizinischen Sterilisationsbehälter (3) oder in einer medizinischen Sterilverpackung.

12. Medizinische Sterilverpackung aufweisend einen mehrlagigen Filter (1) nach Anspruch 1.

## Claims

1. A multilayer filter (1) of a medical sterilization container (3) or a medical sterile packaging, each consisting of a core layer or core sheet (7) made of an aramid fabric as puncture protection, which is fully enclosed by at least one first outer layer or outer sheet (6) made of filter material on one side thereof and by at least one second outer layer or outer sheet (8) made of filter material on the other side thereof.

2. The filter (1) or sterile packaging according to any of the preceding claims, **characterized in that** all layers/sheets (6, 7, 8) of the filter (1) have fluid exchange openings or pores (9) which allow the exchange of fluids, in particular gases, between the environment and the interior of the medical sterilization container (3) or sterile packaging.

3. The filter (1) or sterile packaging according to any of the preceding claims, **characterized in that** the at least one first outer layer/sheet or the at least one first and second outer layers/sheets (6, 8) consists/consist of a material of the group consisting of cellulose or plastic, preferably polypropylene, polyethylene or PTFE.

4. The filter (1) or sterile packaging according to any of the preceding claims, **characterized in that** the at least one first and second outer layers (6, 8) have a larger surface diameter than the core layer (7) and are bonded together at their outer edge surrounding the core layer (7), or **in that** the at least one first and second outer layers (6, 8) are directly bonded to the core layer (7).

5. The filter or sterile packaging according to any of claims 2 to 4, **characterized in that** the fluid exchange openings or pores (9) of the at least one first outer layer (6) or of the at least one first and second outer layers (6, 8) are smaller than the diameter of fibers of the aramid fabric (7).

6. The filter (1) or sterile packaging according to any of claims 1 to 3, **characterized in that** the core layer (7) is coated with a coating material (11) from the group consisting of cellulose, polymer, polypropylene and/or PTFE, and is preferably vaporized.

7. The filter (1) or sterile packaging according to any of the preceding claims, **characterized in that** the core layer (7) and the at least one outer layer (6) or the at least one first and second outer layers (6, 8) has/have fluid exchange openings or pores (9), the fluid exchange openings or pores (9) of the core layer (7) preferably having larger diameters than the fluid exchange openings or pores (7) of the at least one outer layer (6, 8).

8. A method for producing a multilayer filter of a medical sterilization container or for producing a medical sterile packaging, **characterized by** the following method steps:
providing a core layer or core sheet made of aramid fabric as puncture protection,
covering the core layer or core sheet on one side thereof as the inside with at least one first outer layer or outer sheet made of filter material and
covering the core layer or core sheet on the other side thereof as the outside with at least one second outer layer or outer sheet made of filter material, so that the core layer or core sheet is fully enclosed.

9. The method according to claim 8, **characterized in that,** in the event that both sides of the core layer or core sheet are covered, the first and second outer layers are formed from two separate filter discs or membranes, preferably from a material of the group consisting of cellulose or plastic, preferably polypropylene, polyethylene or PTFE, which are directly bonded together at their respective outer edge enclosing the core layer or core sheet.

10. The method according to claim 8, **characterized in that,** in the event that both sides of the core layer or core sheet are covered, the core layer made of aramid fabric is coated, preferably vaporized, with the material of the first and second outer layers, the first and second outer layers preferably consisting of a material of the group consisting of cellulose or plastic, preferably polypropylene, polyethylene or PTFE.

11. Usage of a multilayer filter according to claim 1 in a medical sterilization container (3) or in a medical sterile packaging.

12. A medical sterile packaging comprising a multilayer filter (1) according to claim 1.

## Revendications

1. Filtre multicouche (1) d'un récipient de stérilisation médical (3) ou emballage stérile médical, consistant chacun en une couche centrale ou strate centrale (7) d'un tissu aramide servant de protection contre la perforation, qui se compose d'au moins une première couche extérieure ou strate extérieure (6) constituée d'un matériau filtrant sur un côté et est complètement entouré par au moins une seconde couche extérieure/strate extérieure (8) d'un matériau filtrant de l'autre côté de celle-ci.

2. Filtre (1) ou emballage stérile selon l'une quelconque des revendications précédentes, **caractérisé en ce que** toutes les couches/strates (6, 7, 8) du filtre (1) ont des ouvertures ou pores d'échange de fluide (9) qui permettent l'échange de fluides, en particulier de gaz, entre l'environnement et l'intérieur du récipient de stérilisation médical (3) ou de l'emballage stérile.

3. Filtre (1) ou emballage stérile selon l'une quelconque des revendications précédentes, **caractérisé en ce que** l'au moins une première couche/strate extérieure ou les au moins une première et seconde couches/strates extérieures (6, 8) sont constituées d'un matériau du groupe cellulose ou plastique, de préférence en polypropylène, polyéthylène ou PTFE.

4. Filtre (1) ou emballage stérile selon l'une quelconque des revendications précédentes, **caractérisé en ce que** les au moins une première et seconde couches extérieures (6, 8) ont un diamètre de surface plus grand que la couche centrale (7) et sont collées ensemble sur leur bord extérieur entourant la couche centrale (7), ou que les au moins une première et seconde couches extérieures (6, 8) sont collées directement sur la couche centrale (7).

5. Filtre ou emballage stérile selon l'une quelconque des revendications 2 à 4, **caractérisé en ce que** les ouvertures ou pores d'échange de fluide (9) de l'au moins une première couche extérieure (6) ou des au moins une première et seconde couches extérieures (6, 8) sont plus petites que le diamètre des fibres du tissu aramide (7).

6. Filtre (1) ou emballage stérile selon l'une quelconque des revendications 1 à 3, **caractérisé en ce que** la couche centrale (7) est revêtue, de préférence vaporisée, d'un matériau de revêtement (11) du groupe cellulose, polymère, polypropylène et/ou PTFE.

7. Filtre (1) ou emballage stérile selon l'une quelconque des revendications précédentes, **caractérisé en ce que** la couche centrale (7) et l'au moins une couche extérieure (6) ou les au moins une première et seconde couches extérieures (6, 8) présentent des ouvertures ou pores d'échange de fluide (9), dans lequel les ouvertures ou pores d'échange de fluide (9) de la couche centrale (7) ont de préférence des diamètres plus grands que les ouvertures ou pores d'échange de fluide (9) de l'au moins une couche extérieure (6, 8).

8. Procédé de fabrication d'un filtre multicouche d'un récipient de stérilisation médical ou de fabrication d'un emballage stérile médical, **caractérisé par** les étapes de procédé suivantes :
fourniture d'une couche centrale ou strate centrale constituée d'un tissu aramide comme protection contre la perforation,
recouvrement de la couche centrale ou strate centrale d'un côté comme côté intérieur avec au moins une première couche extérieure ou strate extérieure constituée d'un matériau filtrant et
recouvrement de la couche centrale ou strate centrale de son autre côté comme côté extérieur avec au moins une seconde couche extérieure ou strate extérieure en matériau filtrant, de sorte que la couche centrale ou strate centrale est complètement entourée.

9. Procédé selon la revendication 8, **caractérisé en ce que** dans le cas où les deux côtés de la couche centrale ou strate centrale sont recouverts, les première et seconde couches extérieures sont formées de deux disques filtrants ou membranes séparés, de préférence en un matériau du groupe cellulose ou plastique, de préférence en polypropylène, polyéthylène ou PTFE, qui sont liés directement l'un à l'autre sur leur bord extérieur qui entoure la couche centrale ou strate centrale.

10. Procédé selon la revendication 8, **caractérisé en ce que** dans le cas où les deux côtés de la couche centrale ou strate centrale sont recouverts, la couche centrale en tissu aramide est de préférence revêtue du matériau des première et seconde couches extérieures, les première et seconde couches extérieures étant de préférence constituées d'un matériau du groupe cellulose ou plastique, de préférence en polypropylène, polyéthylène ou PTFE.

11. Utilisation d'un filtre multicouche selon la revendication 1 dans un récipient de stérilisation médical (3) ou dans un emballage stérile médical.

12. Emballage stérile médical comprenant un filtre multicouche (1) selon la revendication 1.
